# EUROPÄISCHE PATENTANMELDUNG

(11) **EP 4 095 123 A1**
(43) Veröffentlichungstag der Anmeldung: **30.11.2022**
(21) Anmeldenummer: 21176570.6
(22) Anmeldetag: 28.05.2021
(51) Int. Cl.: C07C 67/08, C07C 67/54, C07C 67/03, C07C 69/80

(54) **VERFAHREN ZUM AUSSCHLEUSEN DER LEICHTSIEDER BEI DER HERSTELLUNG VON DIALKYLTEREPHTHALATEN ODER DIALKYLPHTHALATEN**

(71) Anmelder: Evonik Operations GmbH, 45128 Essen (DE)
(72) Erfinder: GUBISCH, Dietmar, 46286 Dorsten Rhade (DE); HINSKEN, Ralf Klaus, 46286 Dorsten (DE); KRAFT, Johannes, 53859 Niederkassel (DE); SCHNEIDER, Thomas, 46514 Schermbeck (DE)
(74) Vertreter: Evonik Patent Association

(57) **Zusammenfassung**

Gegenstand der Erfindung ist ein Verfahren zum Ausschleusen der Leichtsieder bei der Herstellung von Dialkylterephthalaten oder Dialkylphthalaten mit C8- bis C11-Alkylresten, wobei die Herstellung unter Verwendung eines C8- bis C11-Alkohols oder einer Mischung von zwei oder mehr C8- bis C11-Alkoholen in einem oder mehreren Reaktoren erfolgt. Die bei der Reaktion gebildeten Leichtsieder, beispielsweise durch Wasserabspaltung von den Alkoholen erhaltene Alkene, werden durch ein effizientes Verfahren abgetrennt und können sich so nicht ansammeln.

## Beschreibung

Die vorliegende Erfindung betrifft ein Verfahren zum Ausschleusen der Leichtsieder bei der Herstellung von Dialkylterephthalaten oder Dialkylphthalaten mit C8- bis C11-Alkylresten, wobei die Herstellung unter Verwendung eines C8- bis C11-Alkohols oder einer Mischung von zwei oder mehr C8- bis C11-Alkoholen in einem oder mehreren Reaktoren erfolgt. Die bei der Reaktion gebildeten Leichtsieder, beispielsweise durch Wasserabspaltung von den Alkoholen erhaltene Alkene, werden durch ein effizientes Verfahren abgetrennt und können sich so nicht ansammeln.

Die Herstellung von Dialkylterephthalaten oder Dialkylphthalaten erfolgt seit vielen Jahren über im Stand der Technik bekannte Verfahren. Technische Bedeutung haben dabei insbesondere die direkte Veresterung der Terephthalsäure oder der Phthalsäure bzw. des entsprechenden Säureanhydrids mit dem jeweiligen Alkohol oder einer Mischung aus mehreren Alkoholen. Letzteres führt zu Mischestern, bei denen die beiden Estergruppen unterschiedliche Alkylreste tragen können. Eine weitere bekannte Möglichkeit zur Herstellung von Dialkylterephthalaten oder Dialkylphthalaten ist die Umesterung von Dimethylphthalat oder Dimethylterephthalat mit dem jeweiligen Alkohol. Beide Verfahren, also sowohl die Veresterung als auch die Umesterung, und die dort notwendigen Bedingungen sind dem Fachmann bekannt, beispielsweise aus den Patentanmeldungen WO 03/029180 A1, WO 2009/095126 A1, EP 3 059 222 A1 und EP 3 059 223 A1.

Bei der Herstellung von Dialkylterephthalaten oder Dialkylphthalaten entstehen häufig aber nicht nur die gewünschten Produkte. Es können sich auch Nebenprodukte bilden, beispielsweise Alkene durch Wasserabspaltung von den eingesetzten Alkoholen. Diese Alkene können gelegentlich zu Verfärbungen und Geruchsbildung führen und sind daher unerwünscht. Diese Nebenprodukte sammeln sich im Reaktionsgemisch an und würden bei der Produktabtrennung zusammen mit den nicht umgesetzten Alkoholen anfallen, sich darin also akkumulieren. Der vom Reaktionsgemisch abgetrennte Alkohol oder das vom Reaktionsgemisch abgetrennte Alkoholgemisch kann und soll schon aus wirtschaftlichen Gründen bei nachfolgenden Veresterungen oder Umesterungen wiederverwendet werden. Bei zu hoher Konzentration der Nebenprodukte muss der abgetrennte Alkohol oder das abgetrennte Alkoholgemisch jedoch komplett verworfen werden, damit sich keine Folgeprobleme in der Veresterung oder Umesterung ergeben.

Die Aufgabe der vorliegenden Erfindung war deshalb die Bereitstellung eines Verfahrens, mit dem die Leichtsieder auf einfache Weise ausgeschleust werden können, ohne große Mengen des abgetrennten Alkohols oder des abgetrennten Alkoholgemischs verwerfen zu müssen. Eine weitere Aufgabe war die Bereitstellung eines Verfahrens, das keinen großen apparativen Aufbau erfordert, sondern sich auch in Bestandsanlagen sehr einfach integrieren lässt.

Die zugrundeliegende Aufgabe der vorliegenden Erfindung konnte durch das hier beschriebene Verfahren zum Ausschleusen der Leichtsieder bei der Herstellung von Dialkylterephthalaten mit C8- bis C11-Alkylresten oder Dialkylphthalaten mit C8- bis C11-Alkylresten erreicht werden. Das entsprechende Verfahren ist in Anspruch 1 beschrieben. Bevorzugte Ausgestaltungen des erfindungsgemäßen Verfahrens sind in den Unteransprüchen angegeben.

Gegenstand der vorliegenden Erfindung ist somit ein Verfahren zum Ausschleusen der Leichtsieder bei der Herstellung von Dialkylterephthalaten mit C8- bis C11-Alkylresten oder Dialkylphthalaten mit C8- bis C11-Alkylresten, wobei die Herstellung jeweils unter Verwendung eines C8- bis C11-Alkohols oder einer Mischung von zwei oder mehr C8- bis C11-Alkoholen in einem oder mehreren Reaktoren erfolgt,
wobei nach Erreichen eines Umsatzes von mindestens 90%, vorzugsweise mindestens 92%, besonders bevorzugt mindestens 95% bei der Herstellung der Dialkylterephthalate oder Dialkylphthalate ein leichter Unterdruck im Bereich von 400 bis 900 mbar absolut in dem oder in den Reaktoren erzeugt wird, wodurch eine Leichtsiederphase des Reaktionsgemisches, die zumindest nicht umgesetzten Alkohol und Reaktionsnebenprodukte umfasst, abdestilliert wird, wobei
der zuerst übergehende Teil der abdestillierten Leichtsiederphase ausgeschleust wird, wobei die Menge des ausgeschleusten Teils der Leichtsiederphase 0,05 bis 8 Gew.-%, vorzugsweise 0,1 bis 4 Gew.-% der gesamten abdestillierten Leichtsiederphase beträgt.

Der erfindungsgemäße Vorteil dieses Verfahrens ist die einfache Ausgestaltung, die keinen besonderen präparativen oder apparativen Aufwand erfordert. Das Ausschleusen und damit Verwerfen des zuerst übergehenden Teils nach Anlegen des Unterdrucks ist in vorhandenen Anlagen problemlos möglich. Eine zusätzliche Destillationskolonne zur Aufarbeitung des nicht umgesetzten Alkohols nach der Abtrennung aus dem Reaktionsgemisch ist deshalb nicht erforderlich. Es ist klar, dass bei dem erfindungsgemäßen Verfahren auch ein Teil des nicht umgesetzten Alkohols oder des nicht umgesetzten Alkoholgemischs in dem zuerst übergehenden Teil der Leichtsiederphase vorhanden ist und dementsprechend ebenfalls verworfen wird. Dies ist im Hinblick auf den andernfalls notwendigen apparativen Aufbau mit einer zusätzlichen Destillationskolonne oder dem Verwerfen der vollständigen Leichtsiederphase bei einer zu hohen Nebenproduktkonzentration zu vernachlässigen.

Das erfindungsgemäße Verfahren umfasst die Herstellung von Dialkylterephthalaten mit C8- bis C11-Alkylresten oder Dialkylphthalaten mit C8- bis C11-Alkylresten. Dies geschieht insbesondere durch Veresterung von Terephthalsäure oder Phthalsäure bzw. Phthalsäureanhydrid oder durch Umesterung von Dimethylphthalat oder Dimethylterephthalat. Bei beiden Verfahren werden C8- bis C11-Alkohole oder einer Mischung von zwei oder mehr C8- bis C11-Alkoholen eingesetzt, um die entsprechenden Ester oder Mischester herzustellen.

Die Veresterung von Terephthalsäure bzw. Terephthalsäureanhydrid oder Phthalsäure bzw. Phthalsäureanhydrid mit C8- bis C11-Alkoholen oder einer Mischung von zwei oder mehr C8- bis C11-Alkoholen ist ein bekanntes Verfahren und bereits im Stand der Technik beschrieben, beispielsweise in der WO 03/029180 A1 oder der WO 2009/095126 A1. Die Veresterung findet üblicherweise in flüssiger Phase statt. Dabei werden die entsprechende Säure oder das entsprechende Säureanhydrid mit dem jeweiligen Alkohol oder dem jeweiligen Alkoholgemisch in Gegenwart eines geeigneten Katalysators umgesetzt, wobei Wasser entsteht. Das bei der Veresterung entstehende Wasser wird vorzugsweise schon während der Reaktion abgetrennt. Da die Reaktionstemperaturen üblicherweise über dem Siedepunkt von Wasser liegen, kann das Wasser oder ein Azeotrop aus Wasser und Alkohol einfach dampfförmig abgetrennt werden. Vorzugsweise wird beim erfindungsgemäßen Veresterungsverfahren Phthalsäure oder Phthalsäureanhydrid eingesetzt.

Die Umesterung von Dimethylphthalat oder Dimethylterephthalat mit C8- bis C11-Alkoholen oder einer Mischung von zwei oder mehr C8- bis C11-Alkoholen ist ebenfalls ein bekanntes Verfahren und bereits im Stand der Technik beschrieben, beispielsweise in der WO 2009/095126 A1. Die Umesterung findet üblicherweise in flüssiger Phase statt. Dabei wird das Dimethylphthalat oder Dimethylterephthalat mit dem jeweiligen Alkohol oder den jeweiligen Alkoholgemischen in Gegenwart eines geeigneten Katalysators umgesetzt. Die Methoxygruppe von Dimethylphthalat oder Dimethylterephthalat wird dabei durch eine dem eingesetzten Alkohol oder einem Alkohol des Alkoholgemischs hinsichtlich der Kohlenstoffanzahl entsprechende Alkoxygruppe ersetzt und es entsteht Methanol. Das bei der Veresterung entstehende Methanol wird vorzugsweise schon während der Reaktion abgetrennt. Da die Reaktionstemperaturen üblicherweise über dem Siedepunkt von Methanol liegen, kann das Methanol einfach dampfförmig abgetrennt werden. Vorzugsweise wird beim erfindungsgemäßen Umesterungsverfahren Dimethylterephthalat eingesetzt.

Der bei der erfindungsgemäßen Veresterung oder erfindungsgemäßen Umesterung eingesetzte Alkohol ist ein C8- bis C11-Alkohol oder eine Mischung von zwei oder mehr C8- bis C11-Alkoholen. Dazu gehören insbesondere 2-Ethylhexanol, Octanol, Isononanol, Nonanol, Isodecanol, Decanol, 2-Propylheptanol, Undecanol oder Mischung von zwei dieser Alkohole. Im Rahmen dieser Erfindung werden bevorzugt 2-Ethylhexanol, Isononanol, 2-Propylheptanol, oder Mischungen aus C9- und C11-Alkoholen eingesetzt. Besonders bevorzugt wird bei der erfindungsgemäßen Veresterung oder erfindungsgemäßen Umesterung Isononanol, 2-Propylheptanol oder Isodecanol eingesetzt.

Die erfindungsgemäße Veresterung oder erfindungsgemäße Umesterung wird vorzugsweise diskontinuierlich durchgeführt. Bei der hier bevorzugten diskontinuierlichen Betriebsweise handelt es sich insbesondere um eine Batchproduktion. Es wird also eine durch das Reaktorvolumen begrenzte Menge hergestellt und dann die Reaktion beendet. Nach Entleerung des Reaktors kann anschließend eine neue Batchproduktion gestartet werden.

Als Reaktoren eigenen sich dem Fachmann bekannte Reaktoren, die für die Synthese von Dialkylterephthalaten mit C8- bis C11-Alkylresten oder Dialkylphthalaten mit C8- bis C11-Alkylresten eingesetzt werden können. Geeignete Reaktoren umfassen insbesondere eine Destillationskolonne, über die das gebildete Wasser oder das gebildete Methanol und nach der Reaktion auch die nicht umgesetzten Alkohole abgetrennt werden. Die Destillationskolonne ist vorzugsweise fest mit dem Reaktor oder den Reaktoren des erfindungsgemäßen Verfahrens verbunden. Der Reaktor stellt dabei quasi den Sumpf der Destillationskolonne dar. Durch die vorherrschenden Reaktionstemperaturen können Wasser oder Methanol schon während der Veresterung oder der Umesterung in die Gasphase übergehen und über die Destillationskolonne abdestilliert werden. Die dadurch verlorene Flüssigkeitsmenge kann durch Zuführen des eingesetzten Alkohols oder der eingesetzten Alkoholmischung ersetzt werden.

Die Temperatur bei der Herstellung der Dialkylterephthalate mit C8- bis C11-Alkylresten oder Dialkylphthalate mit C8- bis C11-Alkylresten ist von verschiedenen Faktoren abhängig, beispielweise von der durchgeführten Reaktionsvariante, beträgt aber vorzugsweise 150 °C bis 260 °C. Dieser Temperaturbereich ist sowohl für die Veresterung als auch für die Umesterung geeignet. Der Druck bei Veresterung und bei der Umesterung liegt vorzugsweise im Bereich von 0,5 bis 10 bar absolut. Temperatur und Druck sind vorzugsweise so einzustellen, dass das entstehende Wasser oder das entstehende Methanol schon während der Reaktion abdestilliert werden kann.

Am Ende der Reaktion liegen die hergestellten Dialkylterephthalate mit C8- bis C11-Alkylresten oder Dialkylphthalate mit C8- bis C11-Alkylresten in einer Reaktionslösung vor, die neben den hergestellten Verbindungen auch zumindest die nicht umgesetzten Alkohole oder die nicht umgesetzte Alkoholmischung umfasst. Wie bereits geschildert können bei der Veresterung oder der Umesterung Nebenprodukte entstehen, die leichter sieden als die eingesetzten Alkohole oder Alkoholmischungen. Die wichtigsten Nebenprodukte sind Alkene, die durch die Abspaltung von Wasser vom eingesetzten Alkohol entstehen. Die jeweiligen Alkene sieden bei den hier eingesetzten Alkoholen immer leichter als die jeweiligen Alkohole, aus denen das Alken entstanden ist.

Die entstandenen Nebenprodukte liegen mit den hergestellten Dialkylterephthalaten mit C8- bis C11-Alkylresten oder Dialkylphthalaten mit C8- bis C11-Alkylresten und den nicht umgesetzten Alkoholen in der Reaktionslösung vor. Die nicht umgesetzten Alkohole werden nach der Reaktion vom entstandenen Reaktionsprodukt abgetrennt, wodurch die gebildeten Dialkylterephthalate mit C8- bis C11-Alkylresten oder Dialkylphthalate mit C8- bis C11-Alkylresten als Rohprodukt zurückbleiben. Die entstandenen Nebenprodukte werden dabei mit den nicht umgesetzten Alkoholen vom entstandenen Reaktionsprodukt abgetrennt, da sie, wie erwähnt, in aller Regel leichter sieden. Die Leichtsiederphase, die zur Erzeugung des Rohprodukts abgetrennt wird, umfasst somit zumindest die nicht umgesetzten Alkohole und die entstandenen Nebenprodukte.

Die nicht umgesetzten Alkohole bzw. die abgetrennte Leichtsiederphase werden üblicherweise in einer nachfolgenden Herstellung von Dialkylphthalaten oder Dialkylterephthalaten eingesetzt, weshalb sich die Nebenprodukte anreichen können, wenn sie nicht abgetrennt würden. Da gerade die Alkene hochreaktive Moleküle sind, können sie in den Folgeproduktionen Probleme bereiten, insbesondere dann, wenn sie sich immer weiter anreichern. Probleme, die damit zusammenhängen sind ein verschlechterter Geruch und/oder eine verschlechterte Färbung.

Die erfindungsgemäße Lösung ist die Ausschleusung eines Teils einer Leichtsiederphase, die durch Anlegen eines leichten Unterdrucks von 400 bis 900 mbar absolut, vorzugsweise 500 bis 800 mbar absolut abdestilliert wird. Der Unterdruck wird jedoch nicht willkürlich angelegt, sondern erst bei Erreichen eines Schwellenwerts, hier des Reaktionsumsatzes. Nach Erreichen eines Umsatzes, insbesondere bis zu eine Stunde nach Erreichen dieses Umsatzes, von mindestens 90%, vorzugsweise von mindestens 92%, besonders bevorzugt von mindestens 95%, wird der erwähnte Unterdruck von 400 bis 900 mbar absolut, vorzugsweise 500 bis 800 mbar absolut angelegt. Dazu könnte der Umsatz schon während der Reaktion überwacht werden. Es ist aber auch möglich, dass nicht der Umsatz, sondern ein einziger anderer oder mehrere andere Parameter während der Reaktion überwacht werden, wobei der oder die Parameter, ggf. nach vorheriger Kalibrierung, einen Rückschluss auf den Umsatz oder allgemeiner den Fortschritt der Reaktion zulassen. Ein Beispiel wäre die Überwachung der Reaktionstemperatur bzw. der Temperatur der Reaktionsmischung, wobei nach Erreichen einer bestimmten Temperatur ggf. nach Ablauf eines bestimmten Zeitintervalls der Unterdruck angelegt wird. Denkbar wäre aber auch die Überwachung des Reaktionsfortschritts mittels Gaschromatographie, mittels einer Online-Analytik (z. B. Infrarotspektroskopie) oder anhand der Säurezahl. Möglich wäre aber auch dass nach einer vorher festgelegten Reaktionszeit der gewünschte Unterdruck angelegt wird. Es sei an dieser Stelle darauf hingewiesen, dass die vorgeschlagene Lösung nicht ausschließt, dass die Reaktion während des Anlegens des Unterdrucks noch bis zu einem fast vollständigen oder vollständigen Umsatz weiterläuft.

Sobald der genannte Schwellenwert erreicht wird, wird der leichte Unterdruck von 400 bis 900 mbar absolut im Reaktor angelegt. Dies kann durch bekannte Apparate oder Maschinen erreicht werden. Ein Beispiel für eine solche geeignete Apparatur ist eine (Vakuum-)Pumpe, mit der der Unterdruck im Reaktor erzeugt wird. Die Temperatur bei Anlegen des Unterdrucks kann der Reaktionstemperatur entsprechen. Erfolgt das Anlegen des Unterdrucks erst mit einer gewissen Verzögerung nach Erreichen des Schwellenwerts des Umsatzes kann die Temperatur auch geringer sein als die Reaktionstemperatur.

Durch den angelegten leichten Unterdruck verdampfen zumindest Teile der leichtsiedenden Komponenten, also die leichtsiedenden Nebenprodukte, aber nur zu geringem Teil die nicht umgesetzten Alkohole oder die nicht umgesetzte Alkoholmischung, und fallen als Leichtsiederphase an. Diese Leichtsiederphase wird dann abdestilliert. Da die leichtsiedenden Nebenprodukte in aller Regel leichter sieden als die nicht umgesetzten Alkohole wird die Leichtsiederphase nach Anlegen des leichten Unterdrucks zunächst überwiegend diese Nebenprodukte umfassen. Deshalb wird der bei der Abdestillation der Leichtsiederphase zuerst übergehende Teil der Leichtsiederphase, also der Teil der Leichtsiederphase, der nach Anlegen des Unterdrucks als erstes abdestilliert wird, ausgeschleust und verworfen. Bezogen auf die Menge der gesamten abdestillierten Leichtsiederphase, die zur Erzeugung des Rohprodukts abgetrennt wird, beträgt die Menge des ausgeschleusten Teils des Leichtsiederphase 0,05 bis 8 Gew.-%, vorzugsweise 0,1 bis 4 Gew.-%. Es ist nicht auszuschließen, dass bei dem Ausschleusen auch nicht umgesetzte Alkohole verloren gehen, was im Hinblick auf die Vorteile des Verfahrens hingenommen wird.

Das Ausschleusen des zuerst übergehenden Teils der Leichtsiederphase kann mit geringem apparativem Aufwand erreicht werden, beispielsweise mit einem geeigneten Ventil. Die Menge des ausgeschleusten Teils der Leichtsiederphase kann dann mit einem geeigneten Durchflussmesser gemessen und das Ausschleusen der gewünschten Menge beendet werden, indem beispielsweise das Ventil geschlossen wird. Die ausgeschleuste Leichtsiederphase kann nach dem Ausschleusen entwässert werden und anschließend stofflich oder thermisch verwertet werden.

Ist der zuerst übergehende Teil der Leichtsiederphase ausgeschleust, wird die Destillation weiterbetrieben, um die restliche Leichtsiederphase abzudestillieren und das Rohprodukt zu erzeugen. Die bei der Destillation übergehende Leichtsiederphase wird dann kondensiert, in einem geeigneten Behälter gesammelt und zu einem Wasserabscheider geführt, um mögliches noch vorhandenes Wasser abzutrennen. Anschließend wird die flüssige Leichtsiederphase, die zu diesem Zeitpunkt fast ausschließlich nicht umgesetzte Alkohole enthält, in einem Behälter gelagert und kann in einem nachfolgenden Verfahren zur Herstellung von Dialkylterephthalaten mit C8- bis C11-Alkylresten oder Dialkylphthalaten mit C8- bis C11-Alkylresten eingesetzt werden. Während der Abtrennung der Leichtsiederphase kann aufgrund des angelegten Unterdrucks insbesondere nach der vollständigen Beendigung der Reaktion die Temperatur absinken.

In einer bevorzugten Ausführungsform der vorliegenden Erfindung wird der Druck nach dem Ausschleusen des zuerst übergehenden Teils der Leichtsiederphase weiter verringert. Dadurch kann die Abtrennung der Leichtsiederphase, die dann zum überwiegenden Teil aus den nicht umgesetzten Alkoholen besteht, beschleunigt werden. Der Druck wird dabei vorzugsweise auf 30 bis 300 mbar absolut, vorzugsweise 50 bis 200 mbar absolut abgesenkt. Für die Temperatur gilt dabei das gleiche wie vorher beschrieben, d. h. vorzugsweise wird die Temperatur bei der Abtrennung der Leichtsiederphase mit der Zeit absinken.

In einer bevorzugten Ausführungsform der vorliegenden Erfindung wird das erfindungsgemäße Verfahren zum Ausschleusen bei der Herstellung eines Dialkylterephthalats oder eines Dialkylphthalats aus der Gruppe, bestehend aus Diisononylphthalat (DINP), Di-(2-propylheptyl)phthalat (DPHP), Diisodecylphthalat (DIDP) Diisononylterephthalat (DINT) und Di(2-ethylhexyl)terephthalat (DOTP), eingesetzt. In einer besonders bevorzugten Ausführungsform wird das Verfahren bei der Herstellung von Diisononylphthalat (DINP) eingesetzt. Erfindungsgemäß ergibt sich dann ein Verfahren zum Ausschleusen der Leichtsieder bei der Herstellung von Diisononylphthalat (DINP) durch Veresterung von Phthalsäure oder Phthalsäureanhydrid mit Isononanol (INA) oder durch Umesterung von Dimethylphthalat (DMT) mit Isononanol in einem oder mehreren Reaktoren,
wobei bei Erreichen eines Umsatzes von mindestens 90%, vorzugsweise mindestens 92%, besonders bevorzugt mindestens 95% bei der Herstellung von DINP ein leichter Unterdruck im Bereich von 400 bis 900 mbar absolut in dem oder in den Reaktoren erzeugt wird, wodurch eine Leichtsiederphase des Reaktionsgemisches, die zumindest nicht umgesetztes Isononanol und Reaktionsnebenprodukte umfasst, abdestilliert wird, dadurch gekennzeichnet, dass
der zuerst übergehende Teil der abdestillierten Leichtsiederphase ausgeschleust wird, wobei die Menge des ausgeschleusten Teils der Leichtsiederphase 0,05 bis 8 Gew.-%, vorzugsweise 0,1 bis 4 Gew.-% der gesamten abdestillierten Leichtsiederphase beträgt.

In diesem Fall können die Reaktionsnebenprodukte Nonene sein, die durch Wasserabspaltung vom Isononanol entstehen. Nonene und Isononanol können durch eine ausreichend große Destillationskolonne recht einfach voneinander getrennt, weshalb es hier unter Umständen möglich ist, den Anteil von Alkohol in der ausgeschleusten Leichtsiederphase gering zu halten.

Die Dialkylterephthalate mit C8- bis C11-Alkylresten oder Dialkylphthalate mit C8- bis C11-Alkylresten, bei deren Herstellung das erfindungsgemäße Verfahren eingesetzt wird, können vorteilhaft als Weichmacher oder Teil einer Weichmacherzusammensetzung in Kunststoffen oder Kunststoffzusammensetzungen, als Zusatz in Farben oder Lacken, in Klebstoffen oder Klebstoffkomponenten, in Dichtungsmassen oder als Lösemittel verwendet werden.

Die Dialkylterephthalate mit C8- bis C11-Alkylresten oder Dialkylphthalate mit C8- bis C11-Alkylresten können auch in Mischungen mit anderen Weichmachern, insbesondere sogenannten Schnellgelierern, als Weichmacher eingesetzt werden. Beispiele für solche andere Weichmacher sind Dialkylterephtahalate oder Dialkylphthalate mit Alkylresten mit weniger als 8 Kohlenstoffatomen, Trialkyltrimellitate oder Glykolbenzoate, sowie auch Citrate oder C8-C10-Monoalkylbenzoate. Der Anteil an den Dialkylterephthalaten mit C8- bis C11-Alkylresten oder Dialkylphthalaten mit C8- bis C11-Alkylresten beträgt dann vorzugsweise 15 bis 95 Gew.-%, besonders bevorzugt 20 bis 90 Gew.-% und ganz besonders bevorzugt 25 bis 85 Gew.-%, wobei sich die Anteile aller vorhandenen Weichmacher zu 100 Gew.-% addieren. Die genannten Zusammensetzungen aus den Dialkylterephthalaten mit C8- bis C11-Alkylresten oder Dialkylphthalaten mit C8- bis C11-Alkylresten und anderen Weichmachern können als Weichmacherzusammensetzung in Kunststoffen und Kunststoffzusammensetzungen, Klebstoffen, Dichtungsmassen, Lacken, Farben, Plastisolen oder Tinten verwendet werden.

Die Kunststoffzusammensetzungen, die die Dialkylterephthalate mit C8- bis C11-Alkylresten oder Dialkylphthalate mit C8- bis C11-Alkylresten enthalten können, können Polymere, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalcohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxylbuttersäure (PHB), Polyhydroxylvaleriansäure (PHV), Polyester, Stärke, Cellulose und Cellulose-Derivate, insbesondere Nitrocellulose (NC), Ethylcellulose (EC), Celluloseacetat (CA), CelluloseAcetat/Butyrat (CAB), Gummi oder Silikone sowie Mischungen oder Copolymere der genannten Polymere oder deren monomeren Einheiten enthalten. Vorzugsweise weisen die erfindungsgemäßen Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf. Besonders bevorzugt ist die Verwendung von PVC.

Bevorzugt enthält die erfindungsgemäße Kunststoffzusammensetzung als PVC-Typ Suspensions-, Masse-, Mikrosuspensions- oder Emulsions-PVC. Bezogen auf 100 Gewichtsteile Polymer enthalten die erfindungsgemäßen Zusammensetzungen vorzugsweise von 5 bis 200, bevorzugt von 10 bis 150 Gewichtsteile an erfindungsgemäßem Weichmacher.

Die Kunststoffzusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl, Gleitmittel, Treibmittel, Kicker, Antioxidanzien, Rheologie-Additive, Lichtschutzmittel oder Biozide.

Die erfindungsgemäßen Kunststoffzusammensetzungen aus den Dialkylterephthalaten mit C8- bis C11-Alkylresten oder Dialkylphthalaten mit C8- bis C11-Alkylresten und den vorgenannten Polymermaterialien können als Kunststoffzusammensetzungen, Klebstoffe, Dichtungsmassen, Lacke, Farben, Plastisole, Kunstleder, Fußbodenbeläge, Unterbodenschutz, Gewebebeschichtungen, Dachmembranen, Tapeten oder Tinten oder zu deren Herstellung verwendet werden.

Mit den Weichmacherzusammensetzungen hergestellte Kunststoffprodukte können beispielsweise Profile, Dichtungen, Lebensmittelverpackungen, Folien, Spielzeug, Medizinartikel, Dachbahnen, Kunstleder, Fußbodenbeläge, Unterbodenschutz, beschichtete Gewebe, Dachmembranen, Tapeten, Kabel und Drahtummantelungen sein. Bevorzugte Anwendungsgebiete aus dieser Gruppe sind Lebensmittelverpackungen, Spielzeug, Medizinartikel, Tapeten, Dachmembranen, Gewebebeschichtungen und Fußbodenbeläge.

## Patentansprüche

1. Verfahren zum Ausschleusen der Leichtsieder bei der Herstellung von Dialkylterephthalaten mit C8- bis C11-Alkylresten oder Dialkylphthalaten mit C8- bis C11-Alkylresten, wobei die Herstellung jeweils unter Verwendung eines C8- bis C11-Alkohols oder einer Mischung von zwei oder mehr C8- bis C11-Alkoholen in einem oder mehreren Reaktoren erfolgt,
wobei nach Erreichen eines Umsatzes von mindestens 90% bei der Herstellung der Dialkylterephthalate oder Dialkylphthalate ein leichter Unterdruck im Bereich von 400 bis 900 mbar absolut in dem oder in den Reaktoren erzeugt wird, wodurch eine Leichtsiederphase des Reaktionsgemisches, die zumindest nicht umgesetzten Alkohol und Reaktionsnebenprodukte umfasst, abdestilliert wird, **dadurch gekennzeichnet, dass**
der zuerst übergehende Teil der abdestillierten Leichtsiederphase ausgeschleust wird, wobei die Menge des ausgeschleusten Teils der Leichtsiederphase 0,05 bis 8 Gew.-% der gesamten abdestillierten Leichtsiederphase beträgt.

2. Verfahren nach Anspruch 1, wobei der leichte Unterdruck bei einem Umsatz von mindestens 92% angelegt wird.

3. Verfahren nach Anspruch 1, wobei der leichte Unterdruck bei einem Umsatz von mindestens 95% angelegt wird.

4. Verfahren nach einem der vorhergehenden Ansprüche, wobei das Anlegen des leichten Unterdrucks bis zu eine Stunde nach Erreichen des Umsatzes erfolgt.

5. Verfahren nach einem der vorhergehenden Ansprüche, wobei die Menge des ausgeschleusten Teils der Leichtsiederphase 0,1 bis 4 Gew.-% der gesamten abdestillierten Leichtsiederphase beträgt.

6. Verfahren nach einem der vorhergehenden Ansprüche, wobei die Reaktionsnebenprodukte Alkene umfassen, die durch den Wasserabspaltung aus den eingesetzten Alkoholen entstehen.

7. Verfahren nach einem der vorhergehenden Ansprüche, wobei der oder die Reaktor(en) eine Destillationskolonne umfassen.

8. Verfahren nach einem der vorhergehenden Ansprüche, wobei die restliche Leichtsiederphase, die die nicht umgesetzten Alkohole umfasst, nach dem Ausschleusen des zuerst übergehenden Teils der Leichtsiederphase abdestilliert wird.

9. Verfahren nach Anspruch 8, wobei der Druck bei der Abdestillation der restlichen Leichtsiederphase weiter verringert wird.

10. Verfahren nach Anspruch 9, wobei der Druck nach der Verringerung 30 bis 300 mbar absolut beträgt.

11. Verfahren nach einem der Ansprüche 8 bis 10, wobei die abdestillierte restliche Leichtsiederphase in einem nachfolgenden Verfahren zur Herstellung von Dialkylterephthalaten mit C8- bis C11-Alkylresten oder Dialkylphthalaten mit C8- bis C11-Alkylresten eingesetzt wird.

12. Verfahren nach einem der vorhergehenden Ansprüche, wobei das Dialkylterephthalat oder das Dialkylphthalat aus der Gruppe, bestehend aus Diisononylphthalat (DINP), Di-(2-propylheptyl)phthalat (DPHP), Diisodecylphthalat (DIDP) Diisononylterephthalat (DINT) und Di(2-ethylhexyl)terephthalat (DOTP), ausgewählt wird.

13. Verfahren zum Ausschleusen der Leichtsieder bei der Herstellung von Diisononylphthalat (DINP) durch Veresterung von Phthalsäure oder Phthalsäureanhydrid mit Isononanol (INA) oder durch Umesterung von Dimethylphthalat (DMT) mit Isononanol in einem oder mehreren Reaktoren,
wobei bei Erreichen eines Umsatzes von mindestens 90% bei der Herstellung von DINP ein leichter Unterdruck im Bereich von 400 bis 900 mbar absolut in dem oder in den Reaktoren erzeugt wird, wodurch eine Leichtsiederphase des Reaktionsgemisches, die zumindest nicht umgesetztes Isononanol und Reaktionsnebenprodukte umfasst, abdestilliert wird, **dadurch gekennzeichnet, dass**
der zuerst übergehende Teil der abdestillierten Leichtsiederphase ausgeschleust wird, wobei die Menge des ausgeschleusten Teils der Leichtsiederphase 0,05 bis 8 Gew.-% der gesamten abdestillierten Leichtsiederphase beträgt.
